Europäisches Patentamt

European Patent Office

Office européen des brevets

(19)

(11) Publication number: **0 272 095**

**A2**

(12) **EUROPEAN PATENT APPLICATION**

(21) Application number: **87311051.4**

(22) Date of filing: **15.12.87**

(51) Int. Cl.4: **C12P 19/18 , C07H 3/06**

(30) Priority: **15.12.86 JP 296615/86**

(43) Date of publication of application:
**22.06.88 Bulletin 88/25**

(84) Designated Contracting States:
**CH DE FR GB IT LI NL SE**

(71) Applicant: **KABUSHIKI KAISHA YAKULT HONSHA**
**1-19, Higashishinbashi 1-chome**
**Minato-ku Tokyo 105(JP)**

(72) Inventor: **Keisuke, Matsumoto K.K. Yakult Honsha**
**1-19, Higashi Shinbashi 1-chome**
**Minato-ku Tokyo(JP)**
Inventor: **Tamura, Natsuko K.K. Yakult Honsha**
**1-19, Higashi Shinbashi 1-chome**
**Minato-ku Tokyo(JP)**
Inventor: **Watanabe, Tsunekazu Kabushiki Kaisha**
**Yakult Honsha 1-19 Higashi Shinbashi**
**1-chome**
**Minato-ku Tokyoinbashi 1-chome**
**Minato-ku(JP)**

(74) Representative: **Ablewhite, Alan James et al**
**MARKS & CLERK 57/60 Lincoln's Inn Fields**
**London WC2A 3LS(GB)**

(54) Method for producing galactooligosaccharide.

(57) A method for producing galactooligosaccharides characterized by reacting lactose with an enzyme to obtain a sugar solution containing galactooligosaccharides expressed by the formula Gal-(Gal)$_n$-Glc (wherein Gal denotes a galactose residue, Glc denotes a glucose residue, and n denotes an integer of 1 to 4), then separating the sugar components in the sugar solution by column chromatography using a strong acid cation exchange resin to collect fractions containing high concentrations of galactooligosaccharide from the eluates from the column. Therefore, this method can efficiently recover galactooligosaccharides of high purity from the sugar solution.

EP 0 272 095 A2

# METHOD FOR PRODUCING GALACTOOLIGOSACCHARIDES

## Background of the Invention

The present invention relates to a method for producing oligosaccharide which functions to promote the growth of bifidobacteria in vivo.

A galactose-glucose type of oligosaccharide produced by the transfer reaction of the $\beta$-galactosyl group of lactose is a principal component of the oligosaccharide of breast milk and is useful as a promoter in the growth of bifidobacteria which are useful bacteria living in the human intestines (Japanese Patent Publication No. 20266/1983).

Such oligosaccharide (also denoted as galactooligosaccharides in this specification) expressed by the formula Gal-(Gal)$_n$-Glc (wherein Gal denotes a galactose residue, Glc denotes a glucose residue, and n denotes an integer of 1 to 4) have been employed in various fields; for example, they have been added to fermented milk or powdered milk for infants.

Examples of methods for producing galactooligosaccharides from lactose include a method in which $\beta$-galactosidase of Aspergillus oryzae is reacted with lactose (the above-described publication), and a method which utilizes yeast of Cryptococcus (Japanese Patent Laid-Open No. 251896/1985). However, neither method can change all the lactose into galactooligosaccharides, but produces a solution of a sugar mixture containing about 20 to 60% of galactooligosaccharides, disaccharides mainly composed of unreacted lactose, and secondarily-produced monosaccharides such as glucose and galactose (this solution is referred to as sugar solution in this specification). It is therefore necessary if one is to obtain galactooligosaccharides having high activity as a promoter in the growth of bifidobacteria to purify the sugar solution, i.e. to separate the galactooligosaccharides in the sugar solution from other saccharides, so as to obtain the galactooligosaccharides with high purity.

A conventional purification method is the activated charcoal method (the above-described publication). This method mainly uses an adsorption partition as a separation mode in which the sugar solution is passed through an activated charcoal column to adsorb saccharides in the sugar solution on the column, monosaccharides and lactose then being removed by eluting them with water or ethanol at a low concentration, and the galactooligosaccharides being obtained from an eluate by eluting it with ethanol at a high concentration. However, this method has a disadvantage in that saccharides are not sufficiently separated with respect to their sizes and, if an attempt is made to obtain galactooligosaccharides with high purity, the yield is extremely poor. In addition, there is a problem in that since ethanol is used, the overall expense increases owing to the use of ethanol, the recovery thereof, and the construction of a distillation apparatus, all of which result in an increase in the purification cost.

A gel filtration method has been practiced in which saccharides are separated according to their molecular size by using a filtration medium gel mainly composed of dextran, cellulose, and polyacrylamide which form a structure of three dimensional linking. However, this method can be only performed on a laboratory scale because the life time of the filter medium gel is short, and the appropriate load per unit amount of resin is small so the ability to separate saccharides greatly deteriorates in an overload state, the gel itself being expensive.

Another general method of separating oligosaccharide is a method which uses a synthetic adsorbent comprising a porous polymer having no ion exchange groups (Japanese Patent Laid-Open No. 130297/1986). In this method, a solution to be treated is supplied to a column filled with the synthetic adsorbent, and the adsorbed saccharides are eluted with water or by a concentration gradient of water and alcohol so that the saccharides are sorted while being eluted according to the differences in the affinities of the saccharides and the adsorbent in order of molecular weight. However, it has been found that even if this method is applied to the purification of a sugar solution, the elution with water alone requires too much time for recovering galactooligosaccharides of trisaccharides or more, the recovery ratio is not satisfactory, and the concentration of recovered saccharides is very low. If an aqueous alcohol solution is used for the elution, the recovery ratio of galactooligosaccharides is increased, but the need to equilibrate the column with water before the next sugar solution is charged makes continuous separation impossible and involves the cost of alcohol. In other words, this method also is inefficient, expensive, and is impractical.

On the other hand, since the sugar solution contains a large amount of unreacted lactose, the efficient recovery of the unreacted lactose and its reuse as a material lactose are desirable for decreasing the production cost of the galactooligosaccharides. However, such a reuse of the recovered lactose has problems in that, if the lactose contains monosaccharides such as glucose and galactose, transfer disaccharides are produced when β-galactosidase is reacted with the lactose, resulting in a reduction in the amount of the galactooligosaccharides produced and a decrease in the reaction rate due to the inhibition of the enzyme reaction by galactose. Although the recovered lactose which is reused must therefore contain monosaccharides in amounts which are as small as possible, employing conventional methods of separating and purifying galactooligosaccharides makes it difficult to simultaneously obtain lactose containing monosaccharides in amounts low enough to be reusable for treatment with β-galactosidase.

## Summary of the Invention

In view of the above-described situation, it is an object of the present invention to provide a method of producing high-quality galactooligosaccharides which comprises a purification process that is capable of recovering high-purity galactooligosaccharides from a sugar solution at a higher level of efficiency than that of conventional methods.

It is another object of the present invention to provide a method of producing galactooligosaccharide which is capable of obtaining high-purity galactooligosaccharide at a low cost by recovering lactose containing monosaccharides in amounts low enough to be usable when β-galactosidase is used and by reusing the recovered lactose as a material lactose, and which is hence more advantageous than conventional methods.

In other words, the present invention comprises a first invention characterized in that a sugar solution containing galactooligosaccharides is obtained by reacting lactose with any enzyme or microorganism which can bring about the transfer reaction from the lactose to galactooligosaccharides, the sugar components contained in the sugar solution then being separated by column chromatography using a strong acid cation exchange resin so that fractions containing the galactooligosaccharides in high concentrations are collected from the eluates of the column, as well as a second invention characterized in that, in the method of the first invention, fractions containing lactose in high concentrations are collected from the eluates of the column and reused as part of a material lactose.

## Brief Description of the Drawings

Fig. 1 is an explanatory view of a cyclic operating method for the separating treatment of saccharides in the production method of the present invention; and

Fig. 2 is a graph of the results obtained by the separating treatment of saccharides in Example 1.

## Detailed Description of the Invention

In the production method of the present invention, galactooligosaccharides are produced from lactose by any desired method. In other words, the lactose is reacted with an enzyme (for example, β - galactosidase) or a microorganism (for example, yeast of Cryptococcus) which can bring about the transfer reaction from the lactose to the galactooligosaccharides to obtain a sugar solution containing the galactooligosaccharide.

A typical example of a method of treating lactose with an enzyme is a method in which the treatment with an enzyme is performed by using β-galactosidase of Aspergillus oryzae at a lactose concentration of 10 to 90% by weight, an enzyme concentration of 1 to 100 units/ml, a pH value of 3 to 8, and a temperature of about 20° C or more. The amounts of monosaccharides such as glucose and galactose and the galactooligosaccharides increase substantially linearly as the reaction progresses, but a considerably complicated change then takes place, and the amount of oligosaccharide is apt to decrease gradually at a certain point. Therefore, the reaction is generally stopped at a certain time when the maximum yield of the oligosaccharide has been obtained. The enzyme reaction can be stopped after the treatment solution has been heated at about 90°C or more for 5 to 10 minutes.

When a microorganism is used, the microorganism is cultured in a medium containing the lactose so that galactooligosaccharides are produced in the medium, and the microorganism is removed to obtain a sugar solution.

Whether an enzyme or a microorganism is used in the transfer reaction, the obtained sugar solution is, if required, subjected to preparatory purifications such as a decoloring treatment using fine coal and a desalting treatment using an ion exchange resin, and, when the concentration of the saccharides is low, the sugar solution is concentrated under reduced pressure, the purification then being performed by column chromatography using a strong acid cation exchange resin.

The strong acid cation exchange resin used in the separating purification of saccharides is preferably one that is suitable for chromatography and has a grain size which is as close to uniform as possible. In addition, a Na-, K-, or Ca-form of resin which is mainly composed of a styrene-divinylbenzene copolymer and has -$SO_3$-groups as exchange groups is particularly preferable. In particular, Na-form resins have an excellent ability to separate saccharides, and are thus most preferable. Examples of strong acid cation exchange resins which are particularly preferable for the production method of the present invention include Unibead UBK-101L and Unibead UBK-530 (two resins produced by Mitsubishi Chemical Industries, Ltd.).

In order to achieve good separation, column chromatography using the above-described ion exchange resins must be performed at the temperatures of a resin column, the sugar solution to be treated, and the water used for elution, which are kept as constant as possible in the range of about 50 to 90°C, preferably, about 60 to 80°C. The concentration of the saccharides in the sugar solution supplied to the column is preferably about 30 to 65% by weight as a concentration higher than this range produces disorder in the separation pattern.

The separation of the components of the sugar solution by chromatography using a strong acid cation exchange resin is based on the molecular size exclusion effect (the effect of eluting firstly a saccharide having a larger molecular size which cannot permeate a crosslinked resin).

The operation of separating is basically performed in accordance with the following method:

(1) A sugar solution is supplied to a column to adsorb saccharides on a resin. The amount of the saccharides adsorbed is preferably about 2 to 20% by volume of the volume of the resin.

(2) Water is passed through the column at a SV of about 0.1 to 2/Hr to elute the saccharides.

(3) Fractions which are firstly eluted and contain high concentrations of galactooligosaccharides are collected from the eluates, then fractions containing the galactooligosaccharides and lactose (mixed fractions of the two components), fractions containing high concentrations of lactose, fractions containing lactose and monosaccharides (mixed fractions of the two components), and fractions containing high concentrations of monosaccharides, which are successively eluted, are collected from the eluates (if not required, fractions of the eluates containing no galactooligosaccharides are omitted).

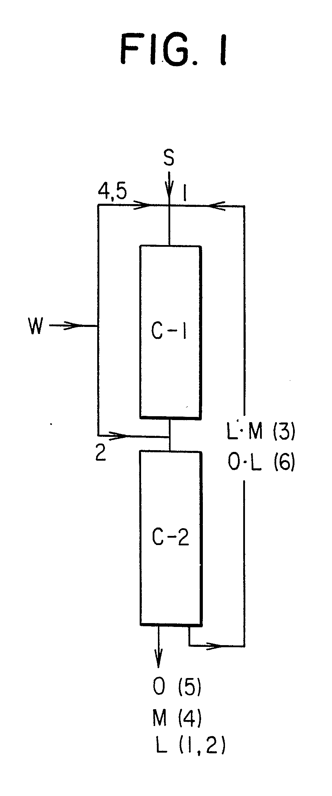

When an attempt is made to obtain high-purity galactooligosaccharides by continuously treating a large amount of sugar solution, it is advantageous to employ the cyclic operating method described below which comprises six steps in one cycle and utilizes two columns C-1, C-2 connected in series, as shown in Fig. 1 (the reference numerals in Fig. 1 denote the step numbers).

Step 1: A sugar solution S is injected into the upper column C-1. At the same time, fractions L containing high concentrations of lactose separated from the previously-injected sugar solution S are eluted from an outlet of the lower column C-2.

Step 2: Water W is injected into the lower column C-2 from the connection between the upper column C-1 and the lower column C-2 so as to accomplish the elution of the fractions L containing high concentrations of lactose from the outlet of the lower column C-2.

Step 3: Fractions L.M containing lactose and monosaccharides (separated from the previously injected sugar solution S) which are eluted from the outlet of the lower column C-2 following the fractions L containing high concentrations of lactose are returned to the upper column C-1.

Step 4: Water W is injected into the upper column C-1 from the inlet thereof to elute fractions M containing high concentrations of monosaccharides (separated from the previously-injected sugar solution S) from the outlet of the lower column C-2.

Step 5: Water W is injected into the upper column C-1 from the inlet thereof to elute fractions O containing high concentrations of the galactooligosaccharides separated from the sugar solution S which was supplied in Step 1 from the outlet of the lower column C-2.

Step 6: Fractions O.L containing the galactooligosaccharides and lactose which are eluted from the outlet of the lower column C-2 following the fractions O containing high concentrations of the galactooligosaccharides are returned to the upper column C-1.

This method can semi-continuously obtain high-purity galactooligosaccharides, lactose, and monosaccharides in the form of a high-concentration eluate which can be easily recovered, and substantially no purification losses of the galactooligosaccharides are caused.

In addition, the fractions containing high concentrations of lactose which are obtained in Steps 1 and 2 can be reused as they are as a material for preparing galactooligosaccharides in the production method of the second invention. When a method using $\beta$-galactosidase which is easily affected by monosaccharides, as described above, is employed as a method of preparing galactooligosaccharides, a problem is the amounts of the monosaccharides in the recovered lactose. However, a reaction material containing

4

monosaccharides in concentrations which do not interfere with the transfer reaction can be easily prepared by selecting the conditions of fractionation so that the concentrations of monosaccharides are sufficiently low, or by using a mixture with lactose which is newly charged. However, if required, the fractions containing high concentrations of lactose may be reused after the monosaccharides have been further removed by way of concentrating the fractions to crystallize lactose.

The method of producing galactooligosaccharide of the present invention has the various advantages described below as compared with conventional methods.

(1) Galactooligosaccharides which have high purity and thus high activity as growth factors for bifidobacteria can be easily produced. In addition, since products containing low concentrations of monosaccharides and lactose can be obtained, this method involves no problems with respect to sweet taste and calorie which would act as obstacles to its use and such that a person with lactose intolerance would be likely suffer from diarrhea, and also facilitates powdering by a freeze-drying or spray-drying method which makes the saccharides easy to handle. It is therefore possible to utilize the excellent properties of galactooligosaccharides in a wider range than has been possible with conventional methods.

(2) Since the strong acid cation exchange resin used in chromatography has excellent resistance to compression, good durability, and superior operating properties, as well as being low-priced, it is suitable for industrial application of this method on a large scale.

(3) The chromatography using the strong acid cation exchange resin can elute with water alone without requiring an organic solvent and a column does not need to be equilibrated with water each time one cycle is completed, unlike a method that uses an adsorbent resin having no ion exchange groups or activated charcoal. Therefore, continuous purification can be easily performed with good efficiency, and the purity and recovery rates of the galactooligosaccharides and lactose are high. In addition, since the expense of recovery and other matters which are necessary with the use of an organic solvent are not required, the running cost is very low.

(4) The recovered lactose has high purity and quality of a level which allows the lactose to often be reused as it is as a reaction material. Therefore, reuse of the lactose enables the production of galactooligosaccharide with a very high yield.

(5) Because of the above-described advantages (2) to (4), it is possible to provide galactooligosaccharide at a very low cost.

(6) The recovered fractions containing high concentrations of monosaccharides can be used as they are as a sweet syrup in the production of food and drinks, and can also be used as a material for sweetening agents.

Examples

The present invention will be described in detail with reference to examples.

Example 1

500 g of pharmaceutical grade lactose was dissolved in 1 l of hot water, a pH 4.5 buffer and 10000 units of $\beta$-galactosidase were added to the obtained solution, and the thus-obtained mixture was subjected to reaction at 40°C for 5 hours. The reaction solution was then heated to 95°C to inactivate the enzyme and filtered, and the filtrate was then passed through a mixed-bed resin tower comprising a strong acid cation exchange resin (Diaion SK1B) and a strong basic anion exchange resin (Diaion PA308) to desalt it. Thereafter, it was concentrated until the concentration of saccharides became 43% by weight. The thus-obtained sugar solution was composed of 24.2% galactooligosaccharide, 43.6% lactose, 23.5% glucose, and 8.7% galactose.

This sugar solution was added to a chromatography column of a strong acid cation exchange resin heat at 60°C (a column with a jacket having an inner diameter of 10 mm and a length of 1000 mm; the resin used, Unibead UBK-101L, Na-form) in an amount of 5% of the amount of the resin, then subjected to elution with hot water at 60°C. The injection speed (SV) of both the sugar solution and the hot water was 0.5/Hr.

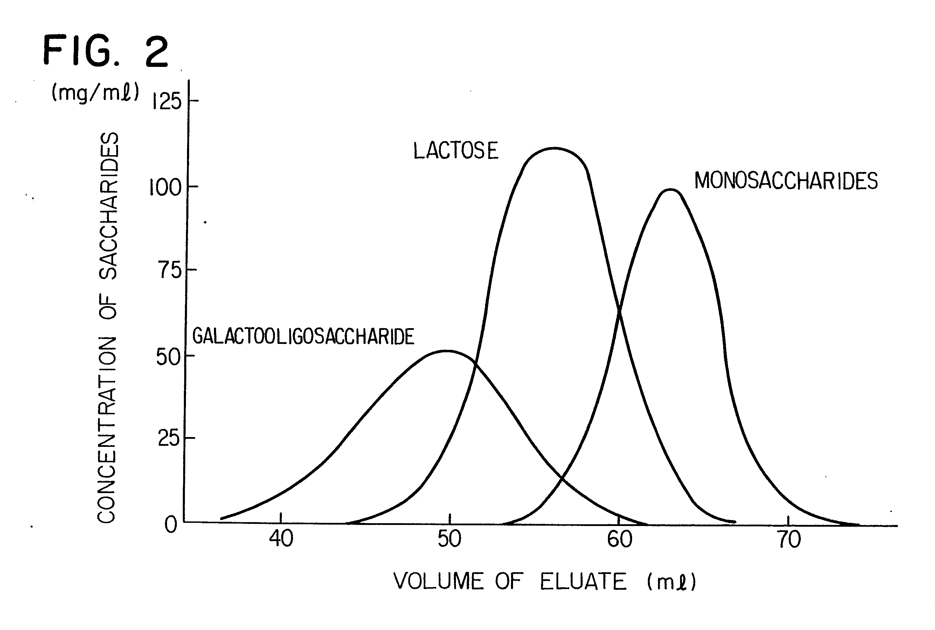

The eluates were collected from the column in 2 ml batches, and the compositions of the saccharides were analyzed by high-performance liquid chromatography. The results are shown in Fig. 2.

Example 2

4 kg of an edible grade lactose was dissolved in 2.3 l of hot water, 80000 units of the same enzyme as that used in Example 1 were added to the solution, and the obtained mixture was subjected to reaction at 67°C for 2 hours. The reaction solution was then heated at 95°C for 10 minutes to inactivate the enzyme, and filtered by adding fine coal and Celite thereto, the filtrate being desalted by using an ion exchange resin. The thus-obtained sugar solution (total concentration of sugar: 62.5%; composition: 29.5% galactooligosaccharide, 45.2% lactose, 25.3% monosaccharides) was continuously treated for 36 cycles of the above-mentioned cyclic operating method in which one cycle comprises six steps, utilizing a chromatography column of a strong acid cation exchange resin heated at 60°C (two stainless-steel columns having an inner diameter of 35.5 mm and a length of 910 mm and connected in series; the resin used: Unibead UBK-530, Na-form, 1810 ml). The amount of the sugar solution charged each time was 120 ml, the temperature of the hot water used for the elution was 60°C, and the injection speed (SV) of both the sugar solution and the hot water was 0.254/Hr.

The compositions of the obtained fractions were as follows:

| | Sugar concentration (% w/w) | Sugar composition (%) | | |
| --- | --- | --- | --- | --- |
| | | Galacto-oligo-saccharide | Lactose | Mono-saccharide |
| Oligo-saccharide fraction | 8.8 | 85.3 | 13.9 | 0.8 |
| Lactose fraction | 24.5 | 18.3 | 77.3 | 4.4 |
| Mono-saccharide fraction | 11.9 | 0 | 2.4 | 97.6 |

The fractions (9 l) containing high concentrations of galactooligosaccharides were concentrated under reduced pressure, and the obtained concentrate was then subjected to freeze drying to obtain 800 g of white galactooligosaccharide powder.

Example 3

Lactose was reacted with β -galactosidase of Aspergillus oryzae in the same manner as Example 1 to obtain a sugar solution composed of 30.1% galactooligosaccharide, 50.0% lactose, and 19.9% monosaccharides. This sugar solution was desalted and decolored by using an ion exchange resin, and 20 l of the obtained sugar solution containing 55% by weight of saccharides in total was then treated by the above-described cyclic operating method using a column (two stainless-steel columns having an inner diameter of 15 cm and a length of 120 cm and connected in series) filled with the same ion exchange resin as that used in Example 2. The amount of the sugar solution charged was 1.87 l/cycle, and fractions containing high concentrations of galactooligosaccharides, fractions containing high concentrations of lactose, and fractions containing high concentrations of monosaccharides were respectively collected from the eluates from the column by treating the sugar solution for 11 cycles and concentrated.

The concentrate of the fractions containing high concentrations lactose (amount of a solid: 8.12 kg; sugar composition: 13.5% galactooligosaccharide, 81.9% lactose, 4.6% monosaccharides) was mixed with 5.88 kg of lactose, and the thus-obtained mixture was then subjected to the enzyme reaction, the preparatory purification, and the separation of saccharides in the same way as that described above.

Such enzyme treatment and separation of saccharides were repeated 10 times, and the concentrate of the obtained fractions containing high concentrations of galactooligosaccharides was passed through a column of a decoloring ion exchange resin to remove a slight color to obtain 66 l of a clear solution containing 50% by weight of sugar. This clear solution was subjected to spray drying to obtian 32 kg of white powder (purity of galactooligosaccharides, 90%) with good fluidity.

On the other hand, a concentrate of the fractions containing high concentrations of monosaccharides (concentration, 70% by weight; purity of monosaccharide, 99%; glucose/galactose = 3/1) was obtained, and this could be used as it was as a mild sweet syrup.

## Claims

(1) A method for producing galactooligosaccharide characterized by reacting lactose with an enzyme or microorganism which can bring about the transfer reaction from said lactose to oligosaccharide expressed by the formula Gal-$(Gal)_n$-Glc (wherein Gal denotes a galactose residue, Glc denotes a glucose residue, and n denotes an integer of 1 to 4) to obtain a sugar solution containing said oligosaccharide, then separating the sugar components in said sugar solution by column chromatography using a strong acid cation exchange resin to collect the fractions containing high concentrations of galactooligosaccharide from the eluates from said column.

(2) A method according to Claim 1, wherein said lactose was reacted with β-galactosidase to produce said oligosaccharide.

(3) A method according to Claim 1, wherein a Na-form of strong acid cation exchange resin which is based on a styrene-divinylbenzene copolymer and has -$SO_3$-groups as exchange groups is used in said column chromatography.

(4) A method for producing galactooligosaccharide characterized by reacting lactose with an enzyme or microorganism which can bring about the transfer reaction from said lactose to oligosaccharide expressed by the formula Gal-$(Gal)_n$-Glc (wherein Gal denotes a galactose residue, Glc denotes a glucose residue, and n denotes an integer of 1 to 4) to obtain a sugar solution containing said oligosaccharide, then separating the sugar components in said sugar solution by column chromatography using a strong acid cation exchange resin to collect fractions containing high concentrations of said galactooligosaccharide from the eluates from said column and to collect fractions containing high concentrations of said lactose therefrom which, and then reusing said fractions containing high concentrations of lactose as part of a material lactose.

(5) A method according to Claim 4, wherein said lactose is reacted with β-galactosidase to obtain said oligosaccharide.

(6) A method according to Claim 5, wherein a Na-form strong acid cation exchange resin which is based on a styrene-divinylbenzene copolymer and has -$SO_3$-groups as exchange groups is used in said column chromatography.

**0 272 095**

# FIG. I

# FIG. 2